(19) Europäisches Patentamt
European Patent Office
Office européen des brevets

(11) **EP 3 915 634 A1**

(12) **EUROPEAN PATENT APPLICATION**

(43) Date of publication:
01.12.2021 Bulletin 2021/48

(51) Int Cl.:
**A61N 5/06** (2006.01)    **A61N 5/067** (2006.01)

(21) Application number: 20176304.2

(22) Date of filing: 25.05.2020

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Fotona d.o.o.**
**1000 Ljubljana (SI)**

(72) Inventors:
• KAZIC, Marko
**1233 Dob pri Domzalah (SI)**
• HRELJAC, Irena
**Ljubljana (SI)**
• LUKAC, Matjaz
**1000 Ljubljana (SI)**

(74) Representative: **Anetsberger, Georg**
**Bardehle Pagenberg Partnerschaft mbB**
**Patentanwälte, Rechtsanwälte**
**Prinzregentenplatz 7**
**DE-81675 München (DE)**

(54) **LASER BRUSH**

(57)    Applicator (100, 200, 300, 600, 700) for ameliorating alopecia, for improving quality, color and density of hair, and/or for improving a condition of a scalp of a person by laser light, comprising means for splitting at least one laser beam (110, 210, 310, 610, 710) into at least two partial laser beams (120, 420, 520, 620, 820) and at least two output means (160, 260, 360, 460, 660, 760), each adapted to apply at least one of the at least two partial laser beams (120, 420, 520, 620, 820) to the person, wherein the at least two output means (160, 260, 360, 460, 660, 760) are adapted such that hair can be accommodated at least partly in between them.

100

Fig. 1

EP 3 915 634 A1

**Description**

1. Technical Field

The present invention relates to an applicator as well as methods for ameliorating alopecia, for improving the quality, color and/or density of hair, and/or for improving a condition of a scalp of a person by laser light.

2. Description of the prior art

Alopecia is very frequent. Particularly, androgenic alopecia affects more than 50% of men and 25% of women over 50 years of age. In men, hair is lost in a well-defined pattern, beginning above both temples. Over time, the hairline recedes to form a characteristic "M" shape, also known as male-pattern baldness. Hair also thins at the crown (near the top of the head), often progressing to partial or complete baldness. The pattern of hair loss in women differs from male-pattern baldness. In women, the hair becomes thinner all over the head, but the hairline does not recede. In women, androgenic alopecia rarely leads to total baldness, but to decreased hair density and thickness. Still, for both men and women, it has a major impact on self-esteem and quality of life.

Androgenic alopecia is a multifactorial process, involving genetic, hormonal and local inflammatory factors. Local fibrosis and degenerative vascular changes are often seen in scalp histologies of persons having androgenic alopecia.

Hair transplantation remains the most effective response to alopecia but comes at considerable cost. More importantly, it is also invasive.

Common pharmaceutical therapies include oral finasteride, an inhibitor of the 5-alpha reductase enzyme, blocking the conversion of testosterone into its more potent form dihydrotestosterone, which is among the determining factors for androgenetic hair loss.

Oral finasteride is available for men only. Other common pharmaceutical therapies rely on oral or topical minoxidil, which is assumed to work by improving the vascularization of the scalp, although its exact mechanism of action remains largely unclear.

It is important to recognize that scalp problems are often at the root of a person's hair concerns. Therefore, improving the condition of the scalp is also of importance. Typical scalp condition related hair concerns include dandruff, and dry and itchy scalp.

Alternative approaches to ameliorating alopecia and stimulating hair growth are based on topical administration of growth factors or natural ointments or on injection of growth factors or platelet rich plasma (PRP). Stem cell therapies are also viable. The common denominator of all these approaches is stimulation of regenerative processes in existing hair follicles and the induction of hair neogenesis.

This effect may also be obtained employing energy-based devices, lasers in particular. Mainly, laser stimulation of hair growth as known in the art uses low-energy lasers of various wavelengths, stimulating cellular metabolism and hence tissue turnover and regeneration (so-called low-level light therapy, or LLLT for short). But it is generally also viable to use higher energy, which may be non-ablative or also ablative, e.g., to drill microholes into tissue, causing mechanical damage resulting in wound-repair induced tissue regeneration (fractional ablative stimulation).

Approaches for ameliorating alopecia usually envisage stimulation of hair growth (e.g. density and/or thickness) in the beginning stages of the condition, when there may still be a substantial amount of hair present. But delivering laser light to skin covered with hair - in particular the scalp - is a challenge. Laser light will be absorbed, reflected and/or scattered by the hair at least partially before reaching the skin. Next to stymieing effectiveness, this also makes determination of accurate, reproducible protocols impossible.

Of course, the most basic solution to this problem is to manually part the hair before applying laser light spot-by spot. This is rather undesirable, though, as it is heavily user-dependent and very time-consuming.

A more elaborate solution known in the art is to arrange laser diodes in rows or other patterns on a comb-like device that parts the hair and thus enables laser light delivery directly to the skin. However, due to the necessary restrictions in size of the laser diodes, only low-energy laser light may be utilized in such devices, restricting this solution to the LLLT domain. Moreover, even with these devices it remains difficult to cover the whole scalp - or any target area more generally - equally.

Therefore, there is a need for an improved approach to ameliorating alopecia, improving quality, color and/or density of hair, and/or improving a condition of a scalp by laser light.

It is to be noted that in the following the expression "ameliorating alopecia" is always meant to additionally also encompass improving quality, color and/or density of hair, and/or improving a condition of a scalp.

3. Summary of the invention

The above need is at least partly met by an applicator according to claim 1.

In a first aspect, the present invention relates to an applicator for ameliorating alopecia, for improving quality, color and/or density of hair, and/or for improving a condition of a scalp of a person by laser light that comprises means for splitting at least one laser beam into at least two partial laser beams as well as at least two output means, each adapted to apply at least one of the at least two partial laser beams to the person, wherein the at least two output means are adapted such that hair can be accommodated at least partly in between them.

By splitting at least one laser beam into at least two partial laser beams which are then applied via a suitable number of output means that may accommodate hair at least partly in between them, applicators according to the present invention allow to deliver laser light, in particular high-energy laser light, with power densities of individual laser pulses delivered to tissue surface higher than 10 W/cm$^2$, preferably higher than 100 W/cm$^2$ and most preferably higher than 1000 W/cm$^2$ (the power density may refer to a peak density of a pulse or an average density of a pulse sequence) directly to a tissue surface of a person having alopecia, whether or not the tissue surface is covered with hair, in an even and seamless manner. However, it is to be appreciated that the invention can also be used in combination with treatments consisting of delivering low power laser radiation, with average power densities over the treatment duration of 0.5 s to 5 s being below 3 W/cm$^2$, preferably below 0.5 W/cm$^2$.

By providing the at least two output means in a manner such that they can accommodate hair in between them, they may displace hair, e.g., guiding it through voids in between the output means, rather than moving or hovering atop the hair when the applicator is moved along, e.g., the scalp of a person. In a way, the output means may thus function like the teeth of a comb or a brush. As such, the present invention avoids a manual parting of the hair, dispensing with cumbersome and time-consuming user intervention. Consequently, the present invention also avoids the spot-by-spot application of laser light known from the state of the art. In effect, this enables good reproducibility as well as control of parameters and results.

At the same time, splitting at least one laser beam into at least two partial laser beams allows to use high-energy lasers for ameliorating alopecia, for improving quality (e.g., thickness), color and/or density of hair, and/or for improving a condition of a scalp. More specifically, the present invention circumvents the need for a plurality of laser light sources, e.g., one per (partial) laser beam, that accordingly need to be limited in size and hence also in power to be arranged in a compact pattern. Instead, a high energy laser beam may be received from an external source, for example, and then split into partial laser beams. As such, the present invention allows to exploit the desirable effects high-energy laser light has been found to have for reversal of hair loss as well as for formation of new hair follicles. The limited tissue response that may be achieved by LLLT thus may be overcome and more effective triggering of keratinocytes may be achieved that may be essential for the activation of signaling pathways that lead to reversal of hair loss as well as to formation of new hair follicles.

In an example, the at least two output means may be specifically adapted to be placed on a tissue surface of the person, thereby augmenting the hair-displacing effect of the output means. This may maximize reproducibility and control of parameters as well as results as it ensures that no hair is present between the output means and the tissue surface of the person.

In an example, at least one of the output means may comprise at least one of a spacer tube, a hollow waveguide and/or an optical fiber. Spacer tubes, hollow waveguides and optical fibers all allow delivering a (partial) laser beam along their length, while shielding the (partial) laser beam from interaction - i.e., absorption, reflection and/or scattering - with hair of the person on which the applicator is used. More specifically, Spacer tubes, hollow waveguides and optical fibers may function akin to teeth of a comb or a brush while allowing to guide (partial) laser beams to their respective ends. Advantageously, spacer tubes, hollow waveguides and optical fibers do not induce any losses in the (partial) laser beams that they guide and shield, thereby further enhancing reproducibility and control of parameters as well as results.

Additionally or alternatively, at least one of the output means may comprise at least one of an output lens and/or a window that is transparent with respect to the at least one laser beam. By providing at least one of the output means with a window, an inner of the applicator may be isolated from the environment, in particular, e.g., from the person and his/her scalp and/or other tissue surface, while still allowing to output the (partial) laser beam(s), preferably directly to the scalp and/or tissue surface. On the one hand, this is desirable for hygiene reasons. On the other hand, this may avoid damage to the applicator, increasing its longevity. Using an output lens may entail similar advantages, but in addition output lenses allow to further control the (partial) laser beam(s). For example, the output lens may (re)focus the (partial) laser beam, or it may adjust its diameter. In particular if the output means comprise spacer tubes, such output lenses may be used to focus the respective partial laser beam within the spacer and to defocus it again when it leaves the spacer tube. Such an output lens may also be adapted to collimate the partial laser beam applied by the output means. All of this may enhance effectiveness, but also reproducibility and control.

In an example, at least one of the output means may be flexible and/or elastic. On the one hand, this may increase comfort for the person on which the applicator is used, especially if the output means are adapted to be placed on a tissue surface of the person. On the other hand, if the output means are flexible and/or elastic, they may better adapt to the target tissue surface, hence increasing effectiveness, reproducibility and control over the (partial) laser beam application even further.

In another example, at least one, more than one but not all, or all of the output means maybe mounted onto a surface with flexible and/or (visco)elastic properties, such as a polymer foam or similar, which may enable adaptation of the output means to the target tissue surface and thus a more comfortable treatment.

In an example, at least a portion of at least one of the output means is releasably attached to the applicator. For example, the output means may comprise a cover, e.g., a single cover for all output means, or a plurality of covers, one per output means, or a mixture of both. Having such covers releasably attached to the applicator may be desirable for hygiene reasons. For example, after contact with a person's skin, such a cover may easily be removed, cleaned, disinfected and reused, or replaced with a new, possibly sterile one, if use of single use covers is desired. But it may also allow to adapt the output means, possibly on an individual basis, enabling an increase in comfort for the person to which the partial laser beams are to be applied. The same holds true if the output means as such are releasably attached to the applicator, either individually or as a whole, or a mixture of both. Also, having at least a portion of at least one of the output means releasably attached to the applicator may facilitate repair of the applicator, thus increasing its longevity.

In some examples, the applicator may be adapted such that the at least two partial laser beams are applied to at least 50%, preferably at least 80%, more preferably at least 90%, most preferably 100% of a target area, when the applicator is moved over the target area along a preferred direction of movement. This may allow seamless coverage of the target area, in turn leading to better, easier to control and more predictable results.

Generally, a target area may be understood throughout this disclosure as an area that is intended to be subjected to laser light. As such, in some examples, a target area may be understood to refer to areas in which a person loses hair. In other examples, a target area may also be understood as an area that is circumscribed by the outermost spots to which partial laser beams have been, or are to be, applied. In yet other examples, a target area may refer to the area to which the laser light of a single partial laser beam or a plurality thereof has been, or is to be, applied.

Additionally or alternatively, the applicator may be adapted such that the at least two partial laser beams are applied with an overlap of at most 50%, preferably at most 20%, more preferably at most 10%, even more preferably at most 1%, most preferably 0%, when the applicator is moved over a target area along a preferred direction of movement. While, generally speaking, simply overlapping the at least two partial laser beams may effectively eliminate any gaps in coverage - or, phrased differently: reduce the chance to "miss a spot" -, overlapping the at least two partial laser beams may at the same time lead to a less even application of the at least two partial laser beams in total, thereby possibly negatively affecting effectiveness and predictability.

Preferably, the applicator may be adapted such that the at least two partial laser beams are applied to at least 50%, preferably at least 80%, more preferably at least 90%, most preferably 100% of a target area as well as such that the at least two partial laser beams are applied with an overlap of at most 50%, preferably at most 20%, more preferably at most 10%, even more preferably at most 1%, most preferably 0%, when the applicator is moved over the target area along a preferred direction of movement. In other words, preferably, the applicator may be moved along a preferred direction of movement such as to provide substantially full coverage of a target area, however without overlapping the partial laser beams. This yields optimal and even coverage and hence maximizes effectiveness and predictability.

In an example, the applicator may comprise an indication of the preferred direction of movement perceivable by a user at least while the at least two partial laser beams are applied to the person. Thereby, it may be ensured that the applicator is indeed - at least substantially - moved along the preferred direction of movement, such that the above-described advantages may be realized.

For example, to achieve the above-mentioned, desired, substantially full coverage of a target area, however without overlapping the partial laser beams, the at least two output means may be arranged in at least one column comprising N output means, with an average distance $l$ between the N output means, the N output means adapted to apply partial laser beams of average diameter $D$. N is an integer (e.g. two, three or more). Then, the preferred direction of movement of the applicator maybe defined by an angle with respect to the at least one column in the range of 0.5 $\alpha$ to 1.5 $\alpha$, preferably 0.8 $\alpha$ to 1.2 $\alpha$, more preferably 0.9 $\alpha$ to 1.1 $\alpha$, most preferably 0.95 $\alpha$ to 1.05 $\alpha$, wherein

$$\sin \alpha = \frac{D}{l} . \alpha$$

may be any angle between 0° and 90°. As will be discussed in further detail below, this ensures that the partial laser beams leave "tracks" on an area to which they are applied that join seamlessly, however without overlapping.

Each output means may apply a partial laser beam of a certain diameter. Such diameter may be a diameter of a circular cross section of a partial laser beam, or it may be an effective diameter in case of a partial laser beam of a non-circular (e.g., hexagonal, elliptic, etc.) cross section, as averaged over the circumference of that cross section. Such cross section may be understood as a cross section of the partial laser beam immediately where the partial laser beam is output from the respective output means. Additionally or alternatively, said cross section may for example also be understood as a cross section of the partial laser beam where the partial laser beam impinges onto a target, as defined, e.g., by a tissue surface of a person if the applicator is applied to the person (e.g. if the applicator and/or the output means are placed onto a tissue surface of the person). In some examples, e.g., if the partial laser beam is collimated, a cross section of the partial laser beam immediately where the partial laser beam is output from the respective output means may be the same as a cross section of the partial laser beam where the partial laser beam impinges onto a target. The above-used term average diameter may then refer to an average over the diameters of the partial laser beams output by the N output means in a column (or all output means of the applicator). In some examples, some or all of the N output means may each apply a single partial laser beam that may comprise the same, i.e., for example a constant, (effective) diameter.

Generally, a diameter and/or a cross section of a partial laser beam may correspond to (or at least be dependent on) a diameter and/or a cross section of a respective output means outputting the partial laser beam.

Throughout this disclosure, distances between output means are to be understood as measured from center to center of the respective output means. The center of an output means may be understood as the geometric center of a cross section of the output means. Such cross section may be understood to be a cross section of the output means immediately where it outputs a partial laser beam. The above-used term average distance may then refer to an average over the pairwise distances between adjacent output means of the N output means in a column (or over all output means of the applicator). In some examples, some or all of the N output means of a column may be arranged such that they comprise a same, i.e., for example a constant, distance to the respective adjacent output means.

In some examples, the at least two output means may be arranged in a plurality of columns and rows essentially perpendicular to each other. Such an arrangement may allow simultaneous application of a larger number of partial laser beams in a predictable manner, hence increasing efficiency without sacrificing predictability. Moreover, each column maybe arranged in the manner just described, such that the partial laser beams corresponding to the output means comprised in the respective column are applied evenly and seamlessly as described above if the applicator is moved along the corresponding preferred direction of movement.

Additionally or alternatively, the distance between the columns of such an arrangement of output means may be chosen in such a way that the areas targeted by different columns of output means also join together substantially seamlessly, but possibly without overlapping, resulting in application of partial laser beams to at least 50%, preferably at least 80%, more preferably at least 90%, most preferably 100% of a target area (and/or an overlap of at most 50%, preferably at most 20%, more preferably at most 10%, even more preferably at most 1%, most preferably 0%), when the applicator is moved over the target area along a preferred direction of movement.

For example, when a first column comprises $N$ output means, with an average distance $l$ between the $N$ output means, the $N$ output means adapted to apply partial laser beams of average diameter $D$, then the first column may be spaced from an adjacent second column of (possibly also $N$) output means by a distance in the range of 0.5 $L$ to 1.5 $L$, preferably 0.8 $L$ to 1.2 $L$, more preferably 0.9 $L$ to 1.1 $L$, most preferably 0.95 $L$ to 1.05

$$L = N \frac{D}{\sqrt{1 - D^2/l^2}}.$$

$L$, wherein $N$ is an integer (e.g. two, three or more). Notably, an applicator with such an arrangement of output means in columns and rows may yield substantially even and seamless coverage of a target area when moved substantially along a preferred direction of movement, which may in this case be defined by an angle with respect to the columns in the range of 0.5 $\alpha$ to 1.5 $\alpha$, preferably 0.8 $\alpha$ to 1.2 $\alpha$, more preferably 0.9 $\alpha$ to 1.1 $\alpha$, most preferably 0.95 $\alpha$ to 1.05 $\alpha$, wherein

$$\sin \alpha = \frac{D}{l}.$$ $\alpha$ may be any angle between 0° and 90°.

More generally, any at least two output means arranged in a two-dimensional pattern of points and adapted to apply partial laser beams of average diameter D may provide such substantially even and seamless coverage of a target area if the two-dimensional pattern of points is obtainable, from a column of points with an average distance $l$ between the points, by translating one or more of the points of the column along a direction having an angle of, e.g. $\alpha$ with respect to the column,

wherein sin $$\sin \alpha = \frac{D}{l}.$$ $\alpha$ It is also within the scope of the present disclosure that the pattern of points is obtainable by translating one or more of the points of the column along a direction having an angle in the range of 0.5 $\alpha$ to 1.5 $\alpha$, 0.8 $\alpha$ to 1.2 $\alpha$, 0.9 $\alpha$ to 1.1 $\alpha$, or 0.95 $\alpha$ to 1.05

$\alpha$, wherein $$\sin \alpha = \frac{D}{l}.$$ $\alpha$ may be any angle between 0° and 90°. The corresponding preferred direction of movement may then correspondingly be defined by an angle with respect to the column of points in the range of 0.5 $\alpha$ to 1.5 $\alpha$, preferably 0.8 $\alpha$ to 1.2 $\alpha$, more preferably 0.9 $\alpha$ to 1.1 $\alpha$, most preferably 0.95 $\alpha$ to 1.05 $\alpha$, or $\alpha$

wherein $$\sin \alpha = \frac{D}{l}.$$

In examples, applicators according to the present invention may further comprise means for receiving the at least one laser beam, in particular from an articulated arm and/or fiber. The means for receiving render applicators according to the present invention particularly versatile as they may hence receive the at least one laser beam from an external source. This is of particular importance because high-energy laser light sources may be too large and unwieldy to be placed within or with the applicator. Also, providing an applicator with means for receiving the at least one laser beam from an external laser light source enables more freedom of design as less parts need to be accommodated within or at the applicator. This freedom may in turn also allow for a more ergonomic design of the applicator, in effect possibly increasing ease of use as well as control. Receiving the at least one laser beam from an articulated arm and/or fiber may further provide for significant freedom of movement, enabling virtually unrestricted movement of the applicator in three-dimensional space. This, too, may allow for a more ergonomic design of the applicator, increasing ease of use and possibly also control.

In another aspect, an applicator as described herein maybe provided with an articulated arm and/or a fiber.

Additionally or alternatively, applicators according to the invention may further comprise beam expanding means. As discussed, the at least one laser beam is split into at least two partial laser beams. In some examples, the at least one laser beam may be expanded beforehand in order to allow for easier spitting. This may become ever more important the more partial laser beams are to be obtained from the at least one laser beam.

Additionally or alternatively, applicators according to the present invention may comprise reflective means, in particular a plurality of mirror segments. Reflective means may be used to (re)direct the at least one laser beam and/or the at least two partial laser beams. This may increase the freedom of design further, potentially enhancing the applicator's ergonomics, increasing ease of use and control. For example, the at least one laser beam may be received from a direction that is not substantially orthogonal to the application area (defined as the area circumscribed by the outermost output means of an applicator), but rather, e.g., substantially perpendicular thereto. This may in fact be desirable as it may allow the user to place a whole hand, or at least large parts thereof, on the applicator, resulting in easier and more controlled handling of the applicator. In fact, e.g., assuming the at least one laser beam is received from an optical fiber or articulated arm, the optical fiber or articulated arm may be easily integrated in a handle of the applicator. In some examples, the reflective means may double as means for splitting the at least one laser beam. For example, if a plurality of mirror segments is used that are positioned and/or oriented differently, different portions of the - possibly expanded - at least one laser beam may be guided in different directions, e.g., towards different output means, hence obtaining at least two partial laser beams by virtue of the reflective means alone.

Additionally or alternatively, applicators according to the present invention may comprise a plurality of apertures, each aperture corresponding to at least one partial laser beam. Such apertures may be used for, or at least in, splitting the at least one laser beam. For example, the at least one laser beam may be expanded and then (re)directed towards the plurality of apertures. The plurality of apertures then transmits at least a part of the at least one laser beam, thus letting at least two partial laser beams pass.

Additionally or alternatively, applicators according to the present invention may also comprise a plurality of lenses, each lens corresponding to at least one partial laser beam. Similar to a plurality of apertures as just discussed, a plurality of lenses may be used for, or at least in, splitting the at least one laser beam. For example, the plurality of lenses may not be contiguous, resulting in gaps in between the lenses, such that the at least one laser beam may be split into at least two partial laser beams when directed towards the plurality of lenses. In addition, and in this respect different from apertures, lenses may also (re)focus and/or collimate the at least one laser beam and/or the at least two partial laser beams, thereby increasing effectiveness, efficiency and control.

In some examples, there may not be a one-to-one correspondence between partial laser beams, output means and apertures or lenses. In some examples, more than one partial laser beam may correspond to a single output means and/or an aperture and/or a lens. In yet other examples, at least one output means and/or an aperture and/or a lens my not correspond to a partial laser beam, e.g., because there are fewer partial laser beams than there are output means and/or apertures and/or lenses. Notably, therefore, the correspondence between partial laser beams, output means and apertures or lenses if expressed as an average may not only be given in terms of an integer, but also by a rational number.

The skilled person readily understands that, thanks to the reversibility of optical paths, the above-discussed means, including the means for splitting the at least one laser beam, may in principle be arranged in any, arbitrary order and combination.

In an example, an applicator according to the present invention may comprise a flat mirror and/or a plurality of hexagonal lenses and/or one or more diffractive optical elements or similar which split a beam essentially like a plurality of hexagonal lenses. For example, the flat mirror may be adapted to direct a laser beam onto the plurality of hexagonal lenses. Using hexagonal lenses may be advantageous as they may be grouped together to be contiguous. If at least one laser beam - for example an expanded one - is then directed towards the plurality of hexagonal lenses, (almost) no laser light, i.e., energy, may be lost as there are no (non-transparent, i.e., "dead") spaces between the lenses. Still, each hexagonal lens of the plurality of hexagonal lenses focuses a portion of the at least one laser beam as directed towards it. The plurality of hexagonal lenses may then pass on at least two partial laser beams, in particular if the plurality of hexagonal lenses each correspond to one of the at least two output means. Using a plurality of hexagonal lenses is advantageous as this may optimize usage of the at least one laser beam, for example along its full diameter, especially assuming its cross section is substantially circular. However, other cross sections, such as square, rectangular or elliptic ones, are conceivable and any sort of lenses, in particular hexagonal lenses, may readily be arranged as to adapt to the respective cross section of the at least one laser beam. This in turn ensures that as much of the energy of the at least one laser beam is funneled into the at least two partial laser beams. Moreover, as using a plurality of hexagonal lenses may allow to fully exploit the at least one laser beam across its - possibly substantially circular - cross section, the remaining applicator and especially its optical components may be quite simple. For example, one may use a flat mirror to (re)direct the at least one laser beam as splitting and (re)focusing will be achieved by means of the hexagonal lenses either way, with limited to no loss.

In another aspect, the present invention relates to methods for ameliorating alopecia, for improving quality, color and/or density of hair, and/or for improving a condition of a scalp of a person by laser light. For example, such methods may comprise the use of an applicator according to the first aspect of the invention, hence entailing all the above-described advantages.

Whether or not using an applicator according to the first aspect of the present invention, a method for ameliorating alopecia, for improving quality, color and/or density of hair, and/or for improving a condition of a scalp of a person by laser light according to a further aspect of the invention may also comprise directing at least one laser pulse comprising a wavelength onto a tissue surface of the person. The wavelength may be chosen to be a wavelength that is highly absorbed by the tissue surface of the person. An applicator for that purpose may comprise a laser source or may receive laser light from an external source. The tissue surface of the person may be a surface of a scalp of the person. The wavelength may be selected from a wide range, e.g., from near infrared to far infrared. In preferred examples, wavelengths that are highly absorbed in water and/or that have a low penetration depth $\delta$ with respect to the tissue surface may be used.

For example, the wavelength of the at least one laser pulse may be between approximately 1.8 micrometers and approximately 11 micrometers, preferably between 2.6 micrometers and 3.2 micrometers or between 9.1 micrometers and 10.2 micrometers. In some examples, the penetration depth $\delta$ maybe smaller than 30 micrometers, preferably smaller than 10 micrometers, and even more preferably smaller than 4 micrometers. In some examples, the penetration depth $\delta$ may be approximated to be 1 micrometer, e.g., for a Er:YAG laser with a wavelength of 2.94 micrometers (or, more generally, for any wavelength between 2.9 micrometers and 3.2 micrometers, or even between 2.6 micrometers and 3.2 micrometers). In other examples, the penetration depth $\delta$ may be approximated to be 15 micrometers, e.g., for a $CO_2$ laser with a wavelength of 10.64 micrometers (or, more generally, for wavelengths between 9 micrometers and 11 micrometers), or the penetration depth $\delta$ may be approximated to be 3 micrometers, e.g., for a Er,Cr:YSGG laser with a wavelength of 2.78 micrometers (or, more generally, for wavelengths between 2.6 micrometers and 2.9 micrometers). In yet other examples, e.g., for a diode or Ho:YAG or Tm:YAG laser with a wavelength between 1.8 micrometers and 2.2 micrometers, the penetration depth $\delta$ may be approximated to be 100 micrometers.

The at least one laser pulse may be delivered individually or in a pulse sequence.

The at least one laser pulse may be provided with a fluence F such that it is ablative (creating microwounds on and/or in the tissue surface and thus stimulating regeneration of the tissue surface and/or hair follicles) or non-ablative with respect to the tissue surface of the person onto which it is directed. As used herein, the term fluence F is defined as $F=E/A_{spot}$, wherein E is an energy of the at least one laser pulse and $A_{spot}$ is a spot size area of the at least one laser pulse at the tissue surface onto which the at least one laser pulse is directed (e.g., the minimum surface in which at least 90% of the energy E of the at least one pulse is deposited). When the fluence F of the at least one laser pulse is increased to reach values above an ablation threshold, the tissue surface is vaporized and/or ablated. The depth of such ablation increases with increasing fluence F.

In some examples, the fluence F of the at least one laser pulse may be chosen to be below the ablation threshold of the tissue surface. For very short pulses, where heat diffusion into a tissue beneath the tissue surface onto which the at least one laser pulse is directed during the pulse is not appreciable (e.g. pulse durations below 10 microseconds), an ablation threshold fluence $F_{thr}$ can be approximately calculated as $F_{thr} \approx H \times \delta$, where $H \approx 1$ J/mm$^3$ is the tissue's specific heat of ablation (i.e. the energy per tissue volume added to generate ablation), and $\delta$ is the penetration depth in the tissue volume (that may be approximated for various laser sources or wavelengths as outlined further above). For example, given a penetration depth $\delta$ of 1 micrometer, e.g., of a Er:YAG laser, the ablation threshold fluence $F_{thr}$ for a very short pulse would be equal to about 0.1 J/cm$^2$.

The ablation threshold becomes higher for longer pulse durations since thermal diffusion deeper into the tissue during a pulse effectively prolongs the at least one laser beam's thermal penetration depth. For example, for typically encountered Er:YAG pulse durations between 50 and 1500 microseconds, the ablation threshold fluence $F_{thr}$ is between 0.5 J/cm$^2$ and 3 J/cm$^2$, resulting in approximately 10-times higher effective specific heat of ablation (H $\approx$ 10 J/mm$^3$). In some, non-ablative examples, the fluence is chosen to be below 0.1 J/cm$^2$ or below 0.5 J/cm$^2$ (particularly for pulse durations of 5 to 1500 microseconds, 5 to 500 microseconds, 50 to 500 microseconds or 50 to 100 microseconds). It may also be chosen below 1 J/cm$^2$ or below 3 J/cm$^2$ (particularly for pulse durations between 50 to 1500 microseconds, 100 to 1500 microseconds, or 500 to 1500 microseconds). In ablative examples, the fluence maybe chosen above the thresholds listed in the preceding sentences, particularly at least 0.5 J/cm$^2$, at least 3 J/cm$^2$, at least 5 J/cm$^2$, at least 10 J/cm$^2$ or at least 50 J/cm$^2$. In another example, the fluence F of the at least one laser pulse may be chosen to be above the ablation threshold, e.g., the fluence F may be selected to be between 1 J/cm$^2$ and 50J/cm$^2$, preferably between 3 J/cm$^2$ and 10 J/cm$^2$.

It is to be noted that for significantly longer pulse durations (of up to 2000 microseconds), heat diffuses deeply into the tissue during a pulse (up to about 50 micrometers). Therefore, the pulse duration has a significant influence on the ablation threshold fluence $F_{thr}$ only

for lasers with a(n optical) penetration depth $\delta$ that is comparable to or below about 50 micrometers.

In a preferred example, laser pulses or pulse sequences - for example the at least one laser pulse discussed above - may be delivered, e.g., by a Er:YAG laser, in a recently disclosed dual tissue regeneration mode, as described in European patent application EP 18172363 which is incorporated by reference herein. This dual tissue regeneration mode comprises very short laser pulses or pulse sequences that create a non-ablative thermal *"needling"* (i.e., triggering) effect, with the laser pulses so short that a laser pulse delivery time and a temperature diffusion time combined are shorter than 900 microseconds. More specifically, an energy delivery time $t_{ed}$ of at least one of such laser pulse, during which a second half of an energy of the laser pulse is delivered, is chosen sufficiently short, so that, given a wavelength and thus a corresponding penetration depth $\delta$ of the laser pulse (e.g. as approximated by the values indicated further above), $t_{ed} + (1/A) (\delta + \sqrt{(2 A\, t_{ed})})^2 < 900$ microseconds, wherein $A = 0.1$ mm$^2$s$^{-1}$. Laser pulses of such characteristics have been found to be particularly effective for ameliorating alopecia, for improving quality, color and/or density of hair, and/or for improving a condition of a scalp. Specific parameters of such pulses as disclosed by European patent application EP 18172363 are an integral part of the present disclosure.

In some examples, the energy delivery time $t_{ed}$ of the at least one laser pulse may be smaller than 600 microseconds, more preferably smaller than 300 microseconds and even more preferably smaller than 100 microseconds. Yet, it may in some examples be desirable if the energy delivery time $t_{ed}$ is not shorter than approximately 100 nanoseconds.

Pulses, such as the pulses in the above example, e.g., the at least one laser pulse discussed further above, may also be delivered within and/or as a pulse sequence S. A repetition time between subsequent pulses of the pulse sequence S, ts, may be shorter than about 200 milliseconds, preferably shorter than 100 milliseconds, most preferably shorter than 50 milliseconds.

Further, a number $N_S$ of pulses of the pulse sequences S may be selected so that the total duration of the pulse sequence S, $t_{tot}$ = Ns x ts, is shorter than 10 seconds, preferably shorter than 3 seconds, most preferably shorter than 0.5 second.

At least some of the laser pulses delivered on the tissue surface, e.g., the at least one laser pulse discussed above, or portions thereof, maybe adapted to comprise a diameter of at least 10 micrometers, for example a diameter from 50 micrometers to 800 micrometers, or from 100 micrometers to 500 micrometers.

In another example, wavelengths that are not absorbed well by water and/or by the tissue surface but rather penetrate deeper into the tissue beneath the tissue surface may be used. Such wavelengths preferably lie in the range from approximately 800 nanometers to approximately 2000 nanometers. With such wavelengths, localized absorption and heating of absorbing targets inside the volume beneath the tissue surface onto which laser pulses, e.g., the at least one laser pulse discussed above, are directed may be achieved.

The concept of selective photothermolysis, which applies to lasers that penetrate below the tissue surface, enables either selective targeting or non-selective heating of the volume beneath the tissue surface onto which laser pulses, such as the at least one laser pulse discussed above, are directed by modifying the laser pulse duration and fluence F.

Upon irradiation of tissue with a suitable, short laser pulse, energy is deposited in an absorbing structure before much heat can be transferred to surrounding tissue by conduction. The resulting temperature rise in an optically and thermally homogenous absorbing structure is thus directly proportional to the absorbed heat, which is in turn proportional to the fluence of the laser pulse, e.g., the fluence F of the at least one laser pulse discussed above, in the target. In general, however, a significant fraction of the absorbed heat may diffuse away from the absorbing structure during laser exposure, which reduces a peak target temperature and impairs a spatial selectivity of the heating, even if the chosen wavelength provides for a selective absorption of laser energy. Therefore, selection of a suitable laser pulse duration, which determines the spatial confinement of absorbed heat in absorbing structures, is very important. Only laser pulse durations that are not significantly longer than about 10 times the thermal relaxation time $\tau$ enable a maximal temperature rise in the targeted absorbing structure. The relaxation time depends on the penetration depth ($\delta$) as $\tau \approx \delta^2/D$ where $D$ is the thermal diffusivity of the tissue (for skin $D \approx 1. \cdot 10^{-7}$ m$^2$/s). For example, for the Er:YAG laser with $\delta \approx 1\text{-}5\ \mu$m, depending on the dryness of the skin, the thermal relaxation time of epidermis is estimated to be in range from 0.01-0.25 milliseconds, and the pulse duration should not be significantly longer than about 0.1 - 2.5 ms. Otherwise, the heat absorbed locally during a (relatively long) pulse duration will flow also to surrounding tissue, such that temperature will not be maximal. Here, the relaxation time $\tau$ represents a time interval in which an amplitude of a hypothetical temperature rise decreases approximately by a factor of 2 (due to, e.g., diffusion of heat into surrounding tissue).

For example, size-dependent targeting of microscopic islets causes them to heat up, causing a temperature gradient and subsequent heat transfer between the microscopic islets and the surrounding tissue. The pulse duration may be selected as a function of the size of one or more microscopic islets.

In an example, an Nd:YAG laser with a wavelength of 1064 nanometers may be used. The penetration depth $\delta$ of an Nd:YAG laser below the skin surface is about 1 cm, meaning it is a relatively deep penetrating laser. This wavelength is absorbed in melanine and may even, under certain circumstances, be used for hair removal when pulse duration and fluence are adjusted to maximally increase a peak target temperature in hair follicles, causing its destruction. In contrast, pulse duration and fluence may also be adapted to either cause fractional heating of microscopic islets under the tissue surface, or to achieve bulk heating of the tissue surface onto which laser pulses are directed. Both effects can stimulate regeneration of tissue and hair follicles. For example, shorter pulses of approximately 0.1 milliseconds to 2 milliseconds with low fluences of approximately 0.5 J/cm$^2$ to approximately 10 J/cm$^2$ may be used for stimulation. In another example, longer pulses or longer pulse sequences (from approximately 50 milliseconds to approximately 10 seconds) with higher fluences (from approximately 20 J/cm$^2$ to approximately 1000 J/cm$^2$) may be used. In both examples above, the power of the laser pulse is below the threshold for reaching the maximum peak temperature (e.g. critical temperature above which a permanent damage occurs) in the hair follicles.

In some examples, multiple laser modes may be used together, e.g., intermittently or concurrently, for example a combination of microwounding using the ablative method discussed above and stimulation using the non-ablative method discussed above.

The described methods may be used in combination with other topical or systemic therapies that could further increase the effect of ameliorating alopecia, improving quality, color and/or density of hair, and/or improving a condition of a scalp of a person. In an example, different growth factors or patient-derived platelet-rich plasma (PRP) may be used. PRP is a concentrated suspension of autologous platelets suspended in a small amount of plasma after centrifugation. Platelets play a fundamental role in hemostasis and are a natural source of growth factors. After preparation, PRP may be subcutaneously injected with several injections covering a target area. Alternatively, in case ablative techniques (e.g. using ablative wavelengths), which result in microwounds on and/or in the tissue surface, are used, PRP could be only topically applied over the target area without the need for injections, as PRP is then delivered to the scalp directly through the laser-induced microwounds.

In yet another aspect, the present invention relates to the use of laser pulses for ameliorating alopecia, improving quality, color and/or density of hair, and/or improving a condition of a scalp of a person. In particular, laser pulses that correspond in one or more or even all respects to the at least one laser pulse discussed above may be used.

## 4. Brief description of the Figures

Possible examples of the present invention will be described in more detail in the subsequent detailed description with reference to the following figures:

Fig. 1:    Schematic cross section of an example of an applicator according to the present invention;

Fig. 2:    Sectional view of an example of an applicator according to the present invention;

Fig. 3:    Sectional view of an example of an applicator according to the present invention;

Fig. 4:    Schematic top/bottom view of output means and/or partial laser beams applied thereby of an example of an applicator according to the present invention;

Fig. 5:    Schematic top/bottom view of partial laser beams applied by output means of an example of an applicator according to the present invention;

Fig. 6:    Schematic cross section of an example of an applicator according to the present invention;

Fig. 7:    Sectional view of an example of an applicator according to the present invention;

Fig. 8A:   Schematic top/bottom view of a plurality of lenses of an example of an applicator according to the present invention;

Fig. 8B:   Schematic top/bottom view of partial laser beams applied by output means of an example of an applicator according to the present invention.

## 5. Detailed description of possible examples

For the sake of brevity only a few examples will be described in the following. The skilled person will recognize that the specific features described with reference to these examples may be modified and combined differently and that individual features may also be omitted if they are not essential. The general explanations in the sections above will also be valid for the following more detailed explanations.

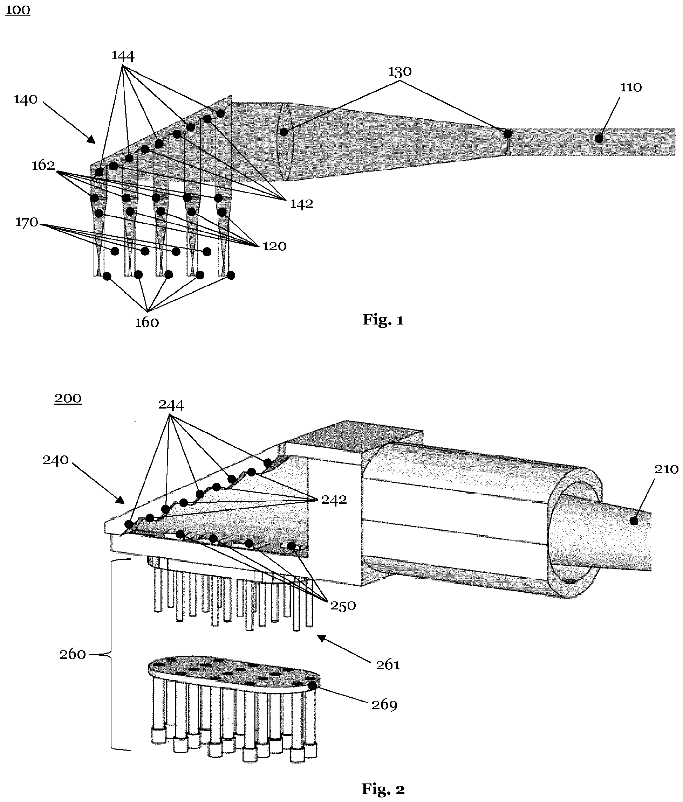

Fig. 1 shows a schematic cross section of an example of an applicator 100 according to the present invention. Therein, a laser beam 110 is received (from the right in Fig. 1).

Laser beam 110 may be collimated. In some examples, laser beam 110 may be received from an articulated arm and/or an optical fiber or similar optical means. In some examples, such an articulated arm or optical fiber may feed laser beam 110 to the applicator from an external laser source (not shown), i.e., from a laser source not arranged within or at the applicator. In particular, the external laser source may be a high-energy laser, with an ability of a power output of more than 0.5 W. Laser beam 110 may be received along a direction essentially parallel to a handle of applicator 100, e.g., horizontally within the drawing plane of Fig. 1.

Applicator 100 may comprise beam expanding means 130, and laser beam 110 may be expanded by means of it. In the example of Fig. 1, beam expanding means 130 comprise two beam-expanding lenses, one (bi)concave, one (bi)convex. However, in other examples, beam expanding means such as beam expanding means 130 may comprise any number of beam-expanding lenses of any shape. In other examples, beam expanding means such as beam expanding means 130 may comprise additional or alternative optical components other than beam-expanding lenses.

After having passed the beam expanding means, laser beam 110 impinges upon reflective means 140 of applicator 100. In the example of Fig. 1, reflective means 140 comprises a plurality of mirror segments 142 and 144 (in some examples, only a single segment 142 maybe provided). Mirror segments 142 and 144 may each comprise a reflective surface having an elongated, rectangular shape. In Fig. 1, this elongated, rectangular shape may extend perpendicular to the drawing plane and/or perpendicular to the direction along which laser beam 110 is received. Mirror segments 142 are oriented substantially parallel to the direction along which laser beam 110 is received. In contrast, mirror segments 144 are angled with respect to laser beam 110. In the example of Fig. 1, mirror segments 144 are angled by substantially 45°, but it is also possible to arrange them at an angle in the range of 20° to 70°, 30° to 60° or 40° to 50°, for example, with respect to the direction along which laser beam 110 is received. Hence, mirror segments 144 (re)direct laser beam 110 by substantially 90°, i.e., mirror segments 144 (re)direct laser beam 110 such as to propagate in a direction that is substantially perpendicular to the direction from which laser beam 110 is initially received. Since mirror segments 142 are oriented substantially parallel to laser beam 110 as it is received, effectively only mirror segments 144 take part in (re)directing laser beam 110 in the example of Fig. 1.

In some examples that comprise mirror segments like mirror segments 142 which are oriented substantially parallel to laser beam 110 as it is received, these mirror segments may not be reflective. In yet other examples, there may be more than two kinds of mirror segments such as mirror segments 142 and 144, each kind arranged at a specific angle with respect to the direction along which laser beam 110 is received.

In the example of Fig. 1, reflective means 140 is sized to - by virtue of at least mirror segments 144 - (re)direct laser beam 110 along its full diameter and/or cross section. In the example of Fig. 1 as well as all subsequent examples, it is assumed for convenience that laser beams such as laser beam 110 comprise a substantially circular cross section. However, the skilled person will realize that the means comprised in the applicator may readily be adapted for use with laser beams such as laser beam 110 of different cross sections, e.g., square, rectangular, hexagonal or elliptic cross sections. In other examples, reflective means such as reflective means 140 may be sized to fully (re)direct laser beams of a larger or smaller diameter and/or cross section than laser beam 110.

Due to the arrangement of mirror segments 142 and 144, laser beam 110 is split into a number of laser beam strips (not shown/labelled) that propagate in a direction that is substantially perpendicular to the direction from which laser beam 110 is initially received. In some examples, the number of resulting laser beam strips may be equal to the number of mirror segments such as mirror segments 144. In the example of Fig. 1, these laser beam strips comprise a substantially rectangular cross section, corresponding to the shape of mirror segments 144. The laser beam strips are spaced apart from each other along the direction from which laser beam 110 is initially received. In the example of Fig. 1, their relative distance corresponds to the size of mirror segments 142, more particularly to the cross-sectional dimension of mirror segments 142 that extends within the drawing plane.

The laser beam strips are (re)directed towards at least two output means 160. Output means 160 may comprise an elongated shape that may extend substantially perpendicular to the direction from which laser beam 110 is initially received. In the example of Fig. 1, output means 160 comprise a substantially cylindrical shape. In other examples, output means such as output means 160 may comprise a different shape, such as a substantially cuboid shape. In the examples of Fig. 1, all output means 160 are substantially the same. However, in other examples, not all output means such as output means 160 may be substantially the same. To the contrary, output means such as output means 160 may differ in size and shape. Sizes and shapes may be varied to increase comfort for the person on which the applicator is used. For example, output means may differ in their length, e.g. one or more output means in the center of the plurality of output means may have a shorter length than one or more output means at a periphery of the output means, such as to adapt to an expected spherical surface of a scalp.

Output means 160 may be arranged to be placed on a surface of a target, e.g. a surface of a scalp. Applicator 100 may comprise an essentially plane lower surface from which each output means 160 protrudes. Output means 160 may comprise a width (understood here with respect to the drawing plane of Fig. 1) in the range of 0.01 centimeter to 2 centimeters, preferably in the range of 0.02 centimeters to 1.5 centimeters, more preferably in the range of 0.05 centimeters to 1 centimeter, most preferably in the range of 0.1 centimeters to 0.5 centimeters. Furthermore, output means may comprise a length in the range of 0.2 centimeters to 10 centimeters, preferably in the range of 0.3 centimeters to 5 centimeters, more preferably in the range of 0.5 centimeters to 3 centimeters, most preferably in the range of 1 centimeter to 2.5 centimeters.

Output means 160 are arranged such that hair can be accommodated at least partly in between them. In the example of Fig. 1, output means 160 define three-dimensional voids 170 in between them to this end. That is, voids 170 are sized as to accommodate hair at least partly. Just as output means 160 themselves, voids such as voids 170 do not need to be the same. Rather, voids such as voids 170 may differ in size and shape. For example, the width (understood here with respect to the drawing plane of Fig. 1) of voids 170 may be in the range of 0.01 centimeter to 10 centimeters, preferably in the range of 0.02 centimeters to 5 centimeters, more preferably in the range of 0,05 centimeters to 2 centimeters, most preferably in the range of 0.1 centimeters to 1 centimeter. The width of voids 170 may correspond to a (average) distance between outer surfaces of two adjacent (e.g., next neighbor) output means 160. Similarly, a height (understood here with respect to the drawing plane of Fig. 1) of voids 170 may correspond to a (average) length of output means 160. For example, a height may be in the range of 0.2 centimeters to 10 centimeters, preferably in the range of 0.3 centimeters to 5 centimeters, more preferably in the range of 0.5 centimeters to 3 centimeters, most preferably in the range of 1 centimeter to 2.5 centimeters.

Output means 160 may be adapted such that laser light is output with a predetermined diameter onto a surface of a target if the applicator is placed onto the surface. In some examples, the laser light may be output as a diverging beam (as shown in Fig. 1). Thus, it may be avoided that the power density applied is too high in case the applicator is incorrectly placed further away from the target than intended by placing it onto the surface of the target. In other examples, a collimated beam may be output.

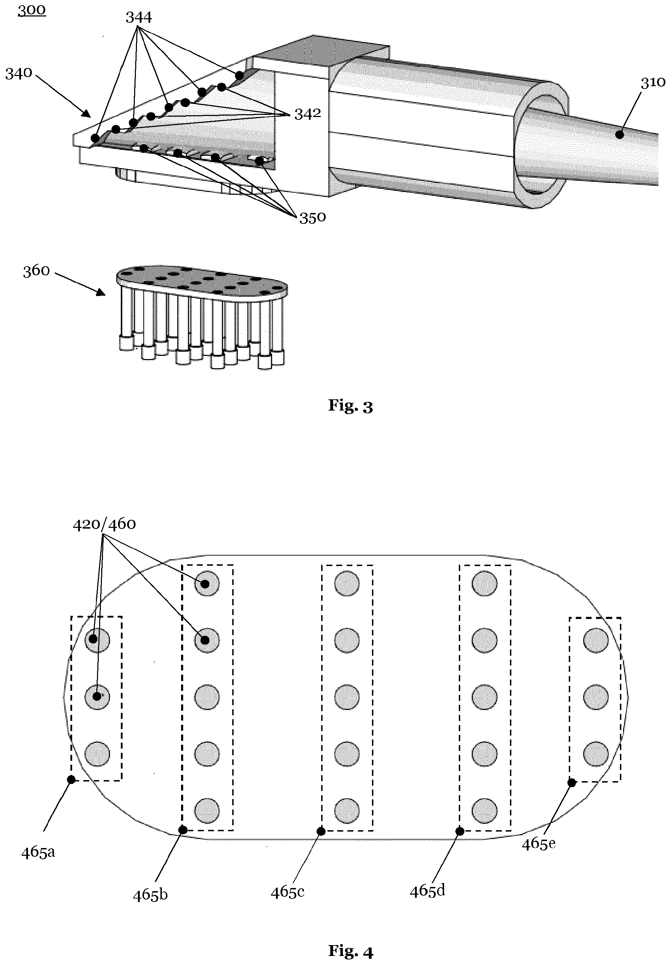

Output means 160 may comprise output lenses 162. Output lenses 162 may focus partial laser beams 120 that are obtained from the laser beam strips (details below in the context of Figs. 2 and 3). Output lenses 162 may also be adapted to collimate partial laser beams 120. In the example of Fig. 1, output lenses 162 are placed closer towards a proximal end of output means 160, i.e., closer to an end through which partial laser beams 120 pass first. In other examples, output means such as output means 160 may each comprise more than one output lens like output lenses 162, or none at all. Also, output lenses such as output lenses 162 may be placed closer towards a distal end of output means 160, i.e., closer to an end through which partial laser beams 120 pass last. Additionally or alternatively, each output means 160 may comprise a window, e.g., at their respective distal ends. This may provide for a well-defined positioning of the output means 160 on the surface of the target and at the same time avoid particles, e.g. dust or dirt particles, from entering the output means 160. The window may be selected from materials that are at least partially transparent to infrared or near-infrared laser wavelengths. These materials can different plastic or thermoplastic polymers, such as polypropylene, polyethylene, polytetrafluoroethylene or similar; or different glass materials, such as, for example sapphire glass or fused quartz glass.

Fig. 2 shows another example of an applicator 200 according to the present invention. Applicator 200 receives a - possibly collimated - laser beam 210 and (re)directs laser beam 210 towards reflective means 240 comprising mirror segments 242 and 244. In these respects, applicator 200 may be considered to be similar to applicator 100, such that the above explanations also apply to applicator 200.

Applicator 200 comprises at least two output means 260. Output means 260 each comprise a core 261 (labelled collectively in Fig. 2) as well as a cover 269 that is releasably attached or attachable, respectively, to applicator 200 and/or cores 261, respectively. In the example of Fig. 2, output means 260 comprise a single, unitary cover 269. In other examples, output means such as output means 260 may comprise a cover 269 consisting of individual portions. For example, a cover such as cover 269 may comprise as many individual portions as there are cores such as cores 261. In the example of Fig. 2, only cores 261 may be adapted to guide partial laser beams (not shown). For example, cores 261 may comprise a hollow waveguide and/or an optical fiber. In contrast, cover 269 may not be adapted to guide partial laser beams, e.g., cover 269 may be opaque with respect to visible light and/or (partial) laser beams. In other examples, also cover 269 may be adapted to guide partial laser beams.

In the example of Fig. 2, cover 269 comprises elongated elements, each adapted to cover a single core 261. The elongated elements comprise a substantially cylindrical shape with a thickened distal end, wherein the distal end of the elongated elements as well as a distal end of cover 269 is defined such that it coincides with the distal end(s) of output means 260 as defined above for output means 160 (being an end through which partial laser beams such as partial laser beams 120 pass last). However, generally, in other examples the shape of such elongated elements and/or a cover such as cover 269 as a whole may be adapted to the shape and size of cores such as cores 261 or to the shape and size of output means such as output means 260 more generally. The thickened distal ends may comprise a different material than a rest of cover 269. For example, the thickened distal ends may comprise a soft material in order to increase comfort for a person on which applicator 200 is used. The distal ends of cover 269 may comprise an optical element, e.g. a transparent window and/or an output lens, which may be adapted to seal the distal ends.

Having output means such as output means 260 comprise a cover such as cover 269 may be advantageous for hygiene reasons. For example, after contact with a person's skin, such a cover may easily be removed, cleaned, possibly disinfected and reused, or it may be replaced with a new, possibly sterile, one.

Applicator 200 may comprise lenses 250. In the example of Fig. 2, lenses 250 are placed right above (upstream with respect to the course of the laser light) output means 260 and below (downstream) reflective means 240. In terms of the optical path of laser beam 210, lenses 250 sit between mirror segments 242 and 244 and output means 260. Each lens 250 may be arranged atop a single output means 260, i.e., one lens 250 corresponds to one output means 260. Lenses 250 may not be arranged contiguously. Instead, they may be arranged in rows that extend perpendicularly to the direction of incoming laser beam 210. In each row, there may be gaps between lenses 250. These gaps may for example be defined by a lower outer wall of applicator 200. The lower outer wall of applicator may be opaque with respect to visible light and/or (partial) laser beams. Hence, the optical path for portions of laser beam 210 impinging thereon is blocked. There may also be gaps between adjacent rows. However, reflective means 240 may be adapted such that essentially the entire incoming laser beam 210 is redirected onto the rows of lenses 250 with essentially no light impinging on the gaps between the rows.

In the example of Fig. 2, laser beam 210 is (re)directed onto reflective means 240. Reflective means 240 splits laser beam 210 into a number of laser beam strips that propagate in a direction that is substantially perpendicular to the direction from which laser beam 210 is initially received, similar as described with respect to laser beam 110 and reflective means 140 of Fig. 1. The laser beam strips impinge on (the rows of) lenses 250 (and the gaps formed between lenses 250 in each row). As the gaps - or the lower outer wall of applicator 200, respectively - are opaque with respect to visible light and/or (partial) laser beams, i.e., block their optical path, the portions of the laser beam strips that impinge onto the gaps do not propagate further, at least not along the direction in which they arrive. Instead, they may be absorbed by the gaps. In contrast, those portions of the laser beam strips that impinge on lenses 250 are guided into output means 260. In the example of Fig. 1, there is a one-to-one correspondence between lenses 250 and output means 260. Preferably, lenses such as lenses 250 are arranged to correspond to reflective means such as reflective means 240. Here, mirror segments 242 and 244 as well as lenses 250 are sized and arranged such that the laser beam strips impinge on corresponding rows of lenses 250, wherein these rows of lenses 250 - just as mirror segments 242 and 244 - extend essentially perpendicularly to the drawing plane. However, generally, lenses such as lenses 250 may be arranged in any, arbitrary pattern.

Fig. 3 shows another example of an applicator 300 according to the present invention. Applicator 300 receives a laser beam 310 and (re)directs laser beam 310 towards a reflective means 340 comprising mirror segments 342 and 344. In these respects, applicator 300 may be considered to be similar or even identical to applicator 200 (and by that virtue also similar to applicator 100), such that the above explanations also apply to applicator 300.

Different from applicator 200, applicator 300 comprises output means 360 (labelled collectively) that are as a whole releasably attached or attachable, respectively, to applicator 300 (as opposed to output means 260 of the example of Fig. 2, of which only a portion, i.e., cover 269, is releasably attached or attachable, respectively, to applicator 200 and/or cores 261, respectively). For example, this may enable to provide applicator 100 with output means 360 of different lengths, e.g. allowing to adapt to different targets, e.g. targets with longer and/or thicker hair or targets with shorter and/or thinner hair.

In some examples, output means such as output means 360 may be releasably attached or attachable, respectively, on an individual basis, i.e., each output means such as output means 360 may be releasably attached or attachable, respectively, independently of other output means 360. In some examples, only some of output means such as output means 360 may be releasably attached or attachable, respectively, either collectively or individually. In yet other examples, none of output means such as output means 360 may be releasably attached or attachable, respectively, neither as a whole nor in portions.

Just as output means 160 of the example of Fig. 1 and output means 260 of the example of Fig. 2, output means 360 are identical in shape and size, but may differ from each other in other examples. For example, the shape and the size, but also the materials of output means 360 may be adapted to provide an increased level of comfort for the person on which applicator 300 is used. For example, they may comprise soft materials and/or may be flexible and/or elastic. Output means 360 may be spacer tubes, i.e., they may consist of a substantially cylindrical outer wall only. But output means 360 may also comprise hollow waveguides or optical fibers. Possibly, output means 360 may also comprise cores (not shown) similar to cores 261 of the example of Fig. 2 which in turn comprise hollow waveguides and/or optical fibers, while also comprising spacer tubes that are arranged around the cores, similar to how cover 269 covers cores 261 in the example of Fig. 2. The skilled person will readily understand that the above may apply to all and any output means discussed herein throughout.

In another example, the proximal end of at least one, more than one but not all, or all of the output means may be attached to and/or enclosed by a base surface comprising flexible and/or (visco)elastic properties, such as a polymer foam or similar. In yet another example, cover 269 may be attached onto such a base surface comprising flexible and/or (visco)elastic properties. This may enable better adaptation of the output means, e.g., to a scalp shape and therefore a more comfortable procedure.

Fig. 4 shows a schematic top and/or bottom view, respectively of output means 460 (not all labelled individually) of an applicator according to the present invention. Notably, for the purposes of the schematic view of Fig. 4, output means 460 may be equated to partial laser beams 420 (not all labelled individually). Output means 460 may be arranged in columns 465a-465e, wherein within each column, the output means may be arranged equidistantly. Columns 465a and 465e comprise fewer output means than columns 465b-465d. Yet, columns 465a and 465e are arranged such that output means 460 comprised therein align with output means 460 comprised in columns 465b-465d. Accordingly, output means 460 are not only arranged in in columns 465a-465e, but simultaneously also in rows, wherein these rows are essentially perpendicular to columns 465a-465e. Overall, the arrangement of output means 460 is therefore symmetric, in particular mirror symmetric. The skilled person will readily understand that partial laser beams 420 may accordingly be applied in a corresponding pattern of columns and rows that are essentially perpendicular to each other. The skilled person will also readily understand that the arrangement of output means 460 illustrated in Fig. 4 is purely exemplary and that other, different arrangements are also possible within the meaning of the present invention. For example, any two-dimensional lattice structure may be used, i.e., output means such as output means 460 may be arranged in a rhombic lattice, square lattice, hexagonal lattice, rectangular lattice, parallelogrammical lattice or triangular lattice. Again, the skilled person will readily understand that partial laser beams such as partial laser beams 420 may then accordingly be applied in a corresponding pattern, i.e., lattice structure. The skilled person understands that this correspondence between arrangement of output means and arrangement of applied partial laser pulses applies throughout the entire present disclosure.

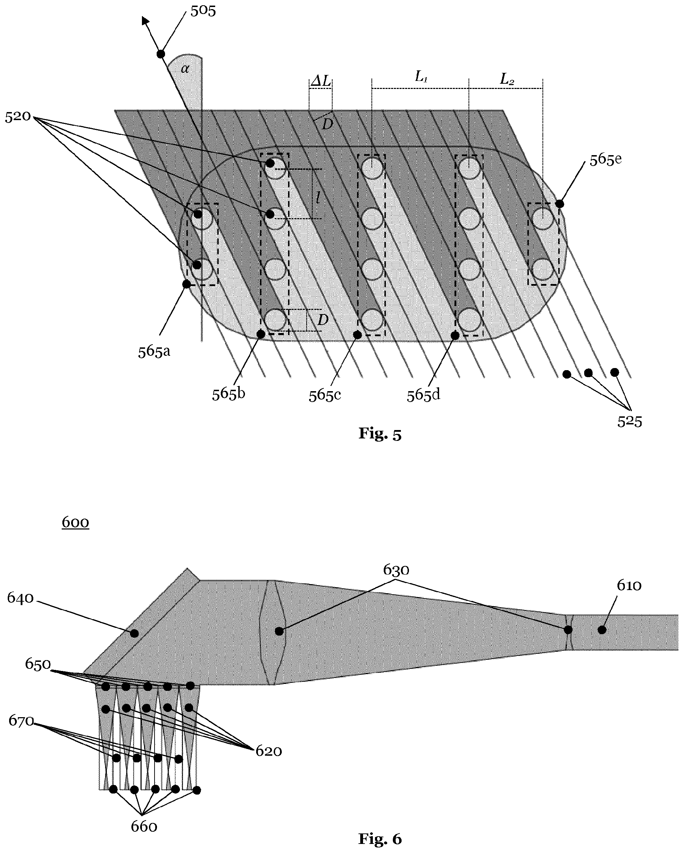

Fig. 5 shows a schematic top and/or bottom view of partial laser beams 520 as they may be applied by output means of an applicator 500 (not shown) according to the present invention. The arrangement of output means applying the shown arrangement of partial laser beams 520 may in general be similar to the one shown in Fig. 4, however with one output means less in every column, such as to correspond to the arrangement of partial laser beams 520 shown in Fig. 5. Notably, partial laser beams 520 may be applied by output means 260 of Fig. 2 or output means 360 of Fig. 3, for example.

Accordingly, partial laser beams 520 are applied in columns 565a-565e. In the example of Fig. 5, columns 565a and 565e comprise two partial laser beams 520, while columns 565b-565d comprise four partial laser beams. Within each column, partial laser beams 520 are spaced apart a distance $l$. All partial laser beams 520 comprise an average diameter $D$. $l$ and $D$ are rational numbers, preferably positive rational numbers. Again, just as laser beams such as laser beams 110, 210 and 310, partial laser beams such as partial laser beams 120, 420 and 520 may comprise a cross section of any shape, e.g., a square, rectangular, elliptic or hexagonal shape. Only for convenience and simplicity, the discussed examples comprise laser beams and partial laser beams of a circular cross section.

Columns 565b and 565c as well as 565c and 565d may be spaced apart a distance $L_1$. $L_1$ is a rational number approximately given by $4\dfrac{D}{\sqrt{1-D^2/l^2}}$ (details below).

Columns 565a and 565b, and columns 565d and 565e are spaced apart a distance $L_2$. $L_2$ is a rational number approximately given by $3\dfrac{D}{\sqrt{1-D^2/l^2}}$ (details below).

As illustrated in Fig. 5, each partial laser beam 520 may be associated with a track 525 (not all labelled). Tracks 525 each denote a respective area to which the respective partial laser beam is applied if applicator 500 is moved along direction 505. As illustrated, direction 505 may be defined by an angle $\alpha$ with respect to the dimension along which columns 565a-565e extend. In other examples, direction 505 may be defined by an angle with respect to the dimension along which columns 565a-565e extend in the range of 0.5 $\alpha$ to 1.5 $\alpha$, preferably 0.8 $\alpha$ to 1.2 $\alpha$, more preferably 0.9 $\alpha$ to 1.1 $\alpha$, most preferably 0.95 $\alpha$ to 1.05 $\alpha$. It holds $\sin\alpha = \dfrac{D}{l}$. $\alpha$ may be any angle between 0° and 90°.

As can be seen, the angle may be selected such that tracks 525 may join (approximately) seamlessly, i.e., the total area to which partial laser beams 520 are applied when applicator 500 is moved along direction 505 is contiguous. At the same time, tracks 525 do not overlap. As such, the arrangement of output means underlying the pattern of partial laser beams 520 of Fig. 5 yields a perfectly even and seamless application of partial laser beams 520. Therefore, direction 505 may be considered a preferred direction of movement.

Direction 505 may be indicated (e.g. on the applicator) such that it is perceivable by a user at least while applicator 500 is in use. For example, direction 505 may be printed onto applicator 500, for example on a backside of applicator 500, or onto any other side with no output means arranged thereon. In other examples, direction 505 may be indicated on a display, e.g., on a computer monitor or a similar digital display, which may either be arranged at applicator 500 or remote therefrom. In yet other examples, direction 505 may be indicated by means of haptic feedback. For example, applicator 500, or at least a handle thereof, could be caused to vibrate in order to notify the user whether he/she is moving applicator 500 along direction 505.

The above factors $4\dfrac{D}{\sqrt{1-D^2/l^2}}$ and $3\dfrac{D}{\sqrt{1-D^2/l^2}}$, respectively, may be derived as follows: Let $\Delta L$ denote a width of a horizontal cut through a single track 525. Horizontal is to be understood with respect to the drawing plane of Fig. 5. Horizontal may additionally or alternatively be understood as perpendicular to the direction along which columns 565a-565e extend. Notably, $\Delta L$ may be different from a width of a track 525, which may be assumed to be given by the average diameter D of a partial laser beam associated with the track 525 as indicated in Fig. 5. A relationship between $\Delta L$ and $D$ may be given by $\cos\alpha = \frac{D}{\Delta L}$, $\alpha$ wherein $\sin\alpha = \frac{D}{l}$ $\alpha$ as above. Recasting this, $\Delta L = \frac{D}{\cos\alpha}$ may be obtained. As is well known, $\sin^2\alpha + \cos^2\alpha = 1$, i.e., $\cos\alpha = \sqrt{1 - \sin^2\alpha}$. Substituting $\sin\alpha = \frac{D}{l}$, $\cos\alpha = \sqrt{1 - D^2/l^2}$ may be obtained. Exploiting this, $\Delta L = \frac{D}{\sqrt{1 - D^2/l^2}}$ may be obtained.

Now to reach the center of a partial laser beam of, e.g., column 565d starting from the center of a corresponding laser beam of, e.g., column 565c, one must traverse approximately the distance $L_1$, or approximately 4 times $\Delta L$. Hence, $L_1 = 4\frac{D}{\sqrt{1 - D^2/l^2}}$. Similarly, to reach the center of a partial laser beam of, e.g., column 565e from the center of a corresponding laser beam of, e.g., column 565d, one must traverse approximately the distance $L2$, or approximately 3 times $\Delta L$. Hence, $L_2 = 3\frac{D}{\sqrt{1 - D^2/l^2}}$. In this context, partial laser beams of different columns may be said to correspond if they may be arranged in a single row that extends perpendicular to the columns.

The pattern of partial laser beams 520 of diameter D shown in Fig. 5, if understood as a pattern of points which each correspond to the center of a respective partial laser beam 520, is an example of a pattern that may be obtained, from a column of points with an average distance $l$ between the points, by translating one or more of the points of the column along a direction having an angle $\alpha$ with respect to the column, wherein $\sin\alpha = \frac{D}{l}$. $\alpha$ may be any angle between 0° and 90°. Adjacent columns obtained in this manner may then approximately be spaced apart a distance $L$ given by $\sin\alpha = \frac{D}{l}$ times the distance by which the respective points have been translated along the direction having the angle $\alpha$ with respect to the column.

Arranging output means of an applicator such as applicator 500 in a manner to provide such a pattern of partial laser beams such as partial laser beams 520 ensures that tracks such as tracks 525 join seamlessly, however without overlapping. That is, arranging output means of an applicator such as applicator 500 in a manner to provide such a pattern of partial laser beams such as partial laser beams 520 ensures that partial laser beams such as partial laser beams 520 cover a target area evenly and seamlessly, i.e., the area on which partial laser beams such as partial laser beams 520 impinge is contiguous.

Fig. 6 shows another example of an applicator 600 according to the present invention. Applicator 600 receives a laser beam 610, expands it using beam expanding means 630 - a pair of beam-expanding lenses, one (bi)convex, on (bi)concave - and (re)directs laser beam 610 towards reflective means 640. In this respect, applicator 600 may be considered to be similar to, e.g., applicators 100, 200 and 300 of Figs 1, 2 and 3, respectively, such that the above explanations also apply to applicator 600.

However, different from applicators 100, 200 and 300 of Figs 1, 2 and 3, respectively, and their respective reflective means 140, 240 and 340, reflective means 640 may not comprise mirror segments such as mirror segments 142, 144, 242, 244, 342, 344. Instead, reflective means 640 may comprise a flat mirror. Reflective means 640 is angled with respect to the direction from which laser beam 610 is initially received by an angle of substantially 45°, but it is also possible to arrange reflective means 640 at an angle in the range of 20° to 70°, 30° to 60° or 40° to 50° with respect to the direction from which laser beam 610 is initially received, for example. Hence, reflective means 640 (re)directs laser beam 610 by substantially 90°, i.e., reflective means 640 (re)directs laser beam 610 such as to propagate in a direction that is substantially perpendicular to the direction from which laser beam 610 is initially received. As reflective means 640 does not comprise mirror segments such as mirror segments 142, 144, 242, 244, 342, 344 of Figs. 1, 2 and 3, respectively, laser beam 610 is not split into laser beam strips, either.

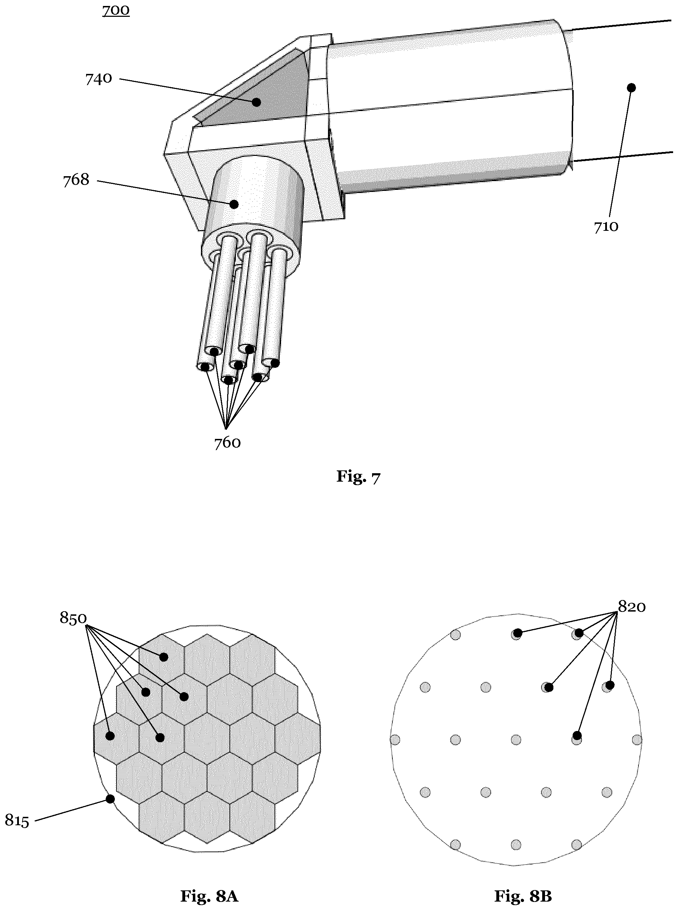

After being (re)directed by reflective means 640, laser beam 610 impinges on an array of lenses 650. In principle, lenses 650 could be similar to lenses 250 and 350 of Figs. 2 and 3, respectively. However, to avoid power loss due to gaps in between lenses 650, lenses 650 may be adapted to be hexagonal, as will be described in greater detail in the context of Fig. 8 (see below). Lenses 650 split laser beam 610 into at least two partial laser beams 620. In fact, lenses 650 may split laser beam 610 in as many partial laser beams 620 as there are lenses 650.

Partial laser beams 620 are guided into output means 660. There may be as many output means 660 as there are lenses 650 and partial laser beams 620. Regarding the shape and size of output means 660 as well as regarding their further characteristics, such as their material composition, it is referred to the above explanations concerning output means 160, 260 and 360 of Figs. 1, 2 and 3, respectively, which apply to output means 660 equally.

In particular, also output means 660 are arranged such that hair can be accommodated at least partly in between them. That is, output means 660 define three-dimensional voids 670 in between them. Voids 670 are sized as to accommodate hair at least partly. Just as output means 660 themselves, voids 670 do not need to be the same. Rather, voids 670 may differ in size and shape.

Fig. 7 shows a further example of an applicator 700 according to the present invention. Applicator 700 receives a laser beam 710. Applicator 700 may be constructed identically to applicator 600, such that the corresponding explanations apply to applicator 700, as well. Therefore, reflective means may be considered to be identical to reflective means 640, and output means 760 may be considered to be identical to output means 660, such that also in this respect the above explanations apply.

As can be gathered from Fig. 7, voids such as voids 670 do not need to extend up to the point where laser beam 710 is split into partial laser beams such as partial laser beams 620. To the contrary, in the example of Fig. 7, lenses 750 (not shown) may be arranged at a proximal end of a common base block 768, i.e., at an end of common base block 768 through which partial laser beams such as partial laser beams 620 pass first. Hence, the partial laser beams are created at the proximal end of common base block 768. However, the voids (not labelled) formed between output means 760 may only extend, e.g., up to the distal end of common base block 768. In the example of Fig. 7, common base block 768 comprises a substantially cylindrical shape. On other examples, common base blocks such as common base block 768 may comprise different shapes, e.g., an elliptical cylindrical - such as in the examples of Figs. 2 and 3 (not labelled there) - or a prism shape. Essentially, the output means 760 protrude from a lower (essentially flat) surface of applicator 700, which may coincide with the lower surface of common base block 768 in the example shown.

In other examples, lenses 750 may also be arranged at a distal end of a common base block such as common base block 768, i.e., at an end of the common base block through which a laser beam such as (re)directed laser beam 710 passes last. Then, the common base block 768 may function like an aperture.

Fig. 8A shows a schematic top and/or bottom view of a plurality of lenses 850 of an example of an applicator according to the present invention. In principle, lenses 850 may correspond to lenses 650 of applicator 600 of Fig. 6, such that the following explanations may apply to the applicator 600 of Fig. 6, as well. Lenses 850 are hexagonal lenses. Moreover, lenses 850 are arranged in a contiguous manner. As shown in Fig. 8A, lenses 850 almost completely cover, or "tile", a cross section 815 of a laser beam that impinges on lenses 850. In principle, said laser beam may correspond to laser beam 610, e.g., after it is (re)directed by reflective means 640. Hence, contiguous hexagonal lenses 850 may allow for a high degree of efficiency, i.e., an amount of energy that is lost when splitting the laser beam into partial laser beams by virtue of lenses 850 is minimized.

Fig. 8B shows a schematic top and or bottom view of partial laser beams 820 applied by output means of an example of an applicator according to the present invention. Partial laser beams 820 may be applied by an applicator employing a plurality of hexagonal lenses such as lenses 850 of Fig. 8A. Centers of partial laser beams 820 may correspond to centers of hexagonal lenses such as lenses 850 that were used in obtaining partial laser beams 820.

The skilled person will understand from the above explanations that the various means discussed herein do not need to be implemented as a single element each. In some examples, certain means may be implemented by a plurality of elements that may or may not simultaneously function as other means or parts thereof, too. For example, while in the example of Fig. 6 lenses 650 may be regarded as means for splitting laser beam 610, in the examples of Figs. 2 and 3 at least mirror segments 244 and 344 together with lenses 250 and 350, respectively, may be regarded as means for splitting laser beam 210 and 310, respectively, although mirror segments 244 and 344 additionally also form part of reflective means 240 and 340, respectively. Yet, one may also solely regard lenses 250 and 350 as means for splitting. Similarly, amongst other things, lenses functioning as means for splitting a laser beam such as lenses 250, 350 or 650 may simultaneously also function as output lenses of output means such as output lenses 162 of output means 160 of the example of Fig. 1.

It is emphasized again that the skilled person will readily understand that all the means discussed herein, including the means for splitting at least one laser beam, may be arranged in any order and combination thanks to the reversibility of optical paths.

The skilled person also understands that wherever reference is made to a definite geometrical relation the respective expressions should be understood to also mean relations that are substantially equal to the definite geometrical relation explicitly referred to, i.e., "perpendicular" should be understood to mean "essentially perpendicular", "angled at 45°" should be understood to mean "angled at substantially 45°", and so forth.

A method for hair scalp regeneration according to the current invention (e.g., for ameliorating alopecia, for improving quality, color and/or density of hair, and/or for improving a condition of a scalp) may comprise any one, two or three of the following three steps either performed separately or as a combination of any of the three steps in any order of succession.

Step 1: Non-ablative thermal stimulation

In one of the embodiments, this step is performed with an Erbium laser with a wavelength between 2.5 and 3.5 $\mu$m, such as for example Er:YAG (2940 nm) or Er,Cr:YSGG (2780 nm). The laser energy is delivered in a pulse train sequence consisting of N subablative laser pulses where N = 1-100, preferably N = 5-40 separated by pulse separation time $t_{ser}$ = 5-500 ms, preferably $t_{ser}$ = 20-250 ms, and characterized by one or more (or all) single laser pulse durations of less than 5 ms, preferably less than 1 ms, and one or more (or all) single pulse fluences $F_o$ below 2.5 J/cm$^2$, preferably below 1 J/cm$^2$, most preferably below 0.5 J/cm$^2$. The cumulative fluence $F_c = N \times F_o$ is for N greater than 1 in the range of 1-50 J/cm$^2$, preferably up to 20 J/cm$^2$ with overall pulse sequence duration $t_c = N \times t_{ser}$ being in the range of 20 ms to 10 s, preferably in the range of 100 ms - 2000 ms. The combination of parameters N, $F_o$ and $t_{ser}$ must be chosen such that the pulse sequence does not result in an appreciable tissue ablation.

Step 2: Ablative resurfacing

Ablative resurfacing may be performed in order to produce small epidermal perforations. The goal of these perforations is to trigger scalp regeneration via tissue healing mechanisms, and/or to facilitate penetration of PRP (Platelet-Rich Plasma) or any other medications intended for the treatment of hair loss and other scalp related indications. For example, the medications may be applied by simply rubbing them thoroughly in, for example, circular motion over the pre-drilled area.
In one of the embodiments this step is performed with an Erbium laser with a wavelength between 2.5 and 3.5 mm, such as for example Er:YAG (2940 nm) or Er,Cr:YSGG (2780 nm). The laser energy may be delivered in N = 1-10 pulses separated by $t_{ser}$ = 20-500 ms, preferably $t_{ser}$ = 50-250 ms, and characterized by one or more (or all) single laser pulse durations of less than 5 ms, preferably less than 1 ms, and one or more (or all) single pulse fluences $F_o$ above 2 J/cm$^2$, preferably above 5 J/cm$^2$, most preferably above 8 J/cm$^2$.

Step 3: Biomodulation

Biomodulation may consist of non-ablative non-thermal (average power density less than 0.2 W/cm$^2$) or minimally thermal (average power density less than 3 W/cm$^2$) stimulation of the scalp.
In one of the embodiments, minimally thermal stimulation is performed with an Nd:YAG laser (1064 nm) with an average power density below 3 W/cm$^2$, preferably below 0.5 W/cm$^2$. The treatment duration may be adjusted from 0.5 s to 5 s to deliver from 0.1 J/cm$^2$ to 1.5 J/cm$^2$.
Other preferred embodiments include the following diode lasers, with the treatment duration adjusted from 0.5 s to 5 s to deliver from 0.1 J/cm$^2$ to 1.5 J/cm$^2$:

1064 nm diode with power density below 0.5 W/cm$^2$ preferably below 0.2 W/cm$^2$;
630-680 nm diode with power density below 0.3 W/cm$^2$ preferably below 0.1 W/cm$^2$;
960-980 nm diode with power density below 0.3 W/cm$^2$ preferably below 0.2 W/cm$^2$.

**Claims**

1. Applicator (100, 200, 300, 600, 700) for ameliorating alopecia, for improving quality, color and/or density of hair, and/or for improving a condition of a scalp of a person by laser light, comprising:

   means for splitting at least one laser beam (110, 210, 310, 610, 710) into at least two partial laser beams (120, 420, 520, 620, 820);
   at least two output means (160, 260, 360, 460, 660, 760), each adapted to apply at least one of the at least two partial laser beams (120, 420, 520, 620, 820) to the person;
   wherein the at least two output means (160, 260, 360, 460, 660, 760) are adapted such that hair can be accommodated at least partly in between them.

2. Applicator (100, 200, 300, 600, 700) according to claim 1, wherein the at least two output means (160, 260, 360, 460, 660, 760) are adapted to be placed on a tissue surface of the person.

3. Applicator (100, 200, 300, 600, 700) according to any of the preceding claims, wherein at least one of the output means (160, 260, 360, 460, 660, 760) comprises at least one of: a spacer tube, a hollow waveguide and/or an optical fiber.

4. Applicator (100, 200, 300, 600, 700) according to any of the preceding claims, wherein at least one of the output means (160, 260, 360, 460, 660, 760) comprises at least one of: an output lens (162) and/or a window that is transparent with respect to the at least one laser beam (110, 210, 310, 610, 710).

5. Applicator (100, 200, 300, 600, 700) according to any of the preceding claims, wherein at least one of the output means (160, 260, 360, 460, 660, 760) is flexible and/or elastic.

6. Applicator (100, 200, 300, 600, 700) according to any of the preceding claims, wherein at least a portion of at least one of the output means (160, 260, 360, 460, 660, 760) is releasably attached to the applicator (100, 200, 300, 600, 700).

7. Applicator (100, 200, 300, 600, 700) according to any of the preceding claims, wherein the applicator (100, 200, 300, 600, 700) is adapted such that the at least two partial laser beams (120, 420, 520, 620, 820) are applied to at least 50%, preferably at least 80%, more preferably at least 90%, most preferably 100% of a target area, when the applicator (100, 200, 300, 600, 700) is moved over the target area along a preferred direction of movement (505).

8. Applicator (100, 200, 300, 600, 700) according to any of the preceding claims, wherein the applicator (100, 200, 300, 600, 700) is adapted such that the at least two partial laser beams (120, 420, 520, 620, 820) are applied with an overlap of at most 50%, preferably at most 20%, more preferably at most 10%, even more preferably at most 1%, most preferably 0%, when the applicator (100, 200, 300, 600, 700) is moved over a target area along a preferred direction of movement (505).

9. Applicator (100, 200, 300, 600, 700) according to claim 7 or 8, further comprising an indication of the preferred direction of movement (505), wherein the indication is perceivable by a user at least while the at least two partial laser beams (120, 420, 520, 620, 820) are applied to the person.

10. Applicator (100, 200, 300, 600, 700) according to any of the claims 7-9, wherein the at least two output means (160, 260, 360, 460, 660, 760) are arranged in at least one column (465a-465e, 565a-565e) comprising N output means (160, 260, 360, 460, 660, 760), with an average distance *l* between the N output means (160, 260, 360, 460, 660, 760), the N output means (160, 260, 360, 460, 660, 760) adapted to apply partial laser beams (120, 420, 520, 620, 820) of average diameter D; and wherein the preferred direction of movement (505) of the applicator (100, 200, 300, 600, 700) is defined by an angle with respect to the at least one column (465a-465e, 565a-565e) in the range of 0.5 $\alpha$ to 1.5 $\alpha$, preferably 0.8 $\alpha$ to 1.2 $\alpha$, more preferably 0.9 $\alpha$ to 1.1 $\alpha$, most preferably 0.95 $\alpha$ to 1.05 $\alpha$, wherein

$$\sin \alpha = \frac{D}{l}.$$

11. Applicator (100, 200, 300) according to any of the preceding claims, wherein the at least two output means (160, 260, 360, 460, 660, 760) are arranged in a plurality of columns (465a-465e, 565a-565e) and rows essentially perpendicular to each other.

12. Applicator (100, 200, 300) according to claim 11, wherein a first column (465a-465e, 565a-565e) comprises N output means (160, 260, 360, 460, 660, 760), with an average distance *l* between the N output means (160, 260, 360, 460, 660, 760), the N output means (160, 260, 360, 460, 660, 760) adapted to apply partial laser beams (120, 420, 520) of average diameter D; and wherein the first column (465a-465e, 565a-565e) is spaced from an adjacent second column (465a-465e, 565a-565e) of output means (160, 260, 360, 460, 660, 760) by a distance in the range of 0.5 *L* to 1.5 *L*, preferably 0.8 *L* to 1.2 *L*, more preferably 0.9 *L* to 1.1 *L*,

$$N \frac{D}{\sqrt{1 - D^2/l^2}}.$$

13. Applicator (100, 200, 300, 600, 700) according to any of the preceding claims, wherein the at least two output means (160, 260, 360, 460, 660, 760) are arranged in a two-dimensional pattern of points and adapted to apply partial laser beams (120, 420, 520, 620, 820) of average diameter *D*, wherein the two-dimensional pattern of points is obtainable, from a column of points with an average distance *l* between the points, by translating one or more of the points of the column along a direction having an angle $\alpha$ with respect to the column, wherein $\sin \alpha = \frac{D}{l}$.

14. Applicator (100, 200, 300, 600, 700) according to any of the preceding claims, wherein the applicator (100, 200, 300, 600, 700) further comprises at least one of:

means for receiving the at least one laser beam (110, 210, 310, 610, 710), in particular from an articulated arm and/or fiber;
beam expanding means (130, 630);
reflective means (140, 240, 340, 640, 740), in particular a plurality of mirror segments (142, 144, 242, 244, 342, 344);
a plurality of apertures, each aperture corresponding to at least one partial laser beam (120, 420, 520, 620, 820); and/or
a plurality of lenses (250, 350, 650, 850), each lens (250, 350, 650, 850) corresponding to at least one partial laser beam (120, 420, 520, 620, 820).

15. Applicator (600, 700) according to any of the preceding claims, wherein the applicator (600, 700) comprises a flat mirror (640, 740) and/or a plurality of hexagonal lenses (650, 850).

16. Applicator (100, 200, 300, 600, 700) according to any of the preceding claims, wherein the at least one laser beam (110, 210, 310, 610, 710) is a high-energy laser beam.

17. Method for ameliorating alopecia, for improving quality, color and/or density of hair, and/or for improving a condition of a scalp of a person by laser light, comprising the use of an applicator (100, 200, 300, 600, 700) according to any of the preceding claims.

18. Method for ameliorating alopecia, for improving quality, color and/or density of hair, and/or for improving a condition of a scalp of a person by laser light, comprising:

   directing at least one laser pulse comprising a wavelength onto a tissue surface of the person, wherein an energy delivery time $t_{ed}$ of the at least one laser pulse, during which a second half of the pulse energy is delivered, is chosen sufficiently short, so that, given the wavelength and thus a corresponding penetration depth $\delta$ of the at least one laser pulse, $t_{ed} + (1/A) (\delta + \sqrt{(2 A t_{ed})})^2 < 900$ microseconds, wherein $A = 0.1$ $mm^2 s^{-1}$.

19. Method according to claim 18, wherein the wavelength is in the range of 2.6 micrometers to 3.2 micrometers, and/or wherein a fluence F of the at least one laser pulse is chosen to be below the ablation threshold, preferably between 0.01 $J/cm^2$ and 3 $J/cm^2$, between 0.05 and 2 $J/cm^2$, between 0.1 and 1.5 $J/cm^2$.

20. Method according to claim 18, wherein the wavelength is in the range of 800 nanometers to 1200 nanometers, and/or wherein a fluence F of the at least one laser pulse is chosen to be below the ablation threshold, namely between 0.5 $J/cm^2$ to 10 $J/cm^2$ or between 20 $J/cm^2$ and 1000 $J/cm^2$.

21. Method according to claim 18, wherein the wavelength is in the range of 1.8 micrometers to 11 micrometers, preferably 0.8 micrometers to 2 micrometers or 9.1 micrometers to 10.2 micrometers.

22. Method according to any of the claims 18-21, further comprising the use of an applicator (100, 200, 300, 600, 700) according to any of the claims 1-16.

100

**Fig. 1**

200

**Fig. 2**

**Fig. 3**

**Fig. 4**

**Fig. 5**

600

**Fig. 6**

700

740

768

710

760

**Fig. 7**

850

820

815

**Fig. 8A**

**Fig. 8B**

Europäisches
Patentamt

European
Patent Office

Office européen
des brevets

# EUROPEAN SEARCH REPORT

Application Number

EP 20 17 6304

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 303 722 A (GODFREY ROBIN E [GB] ET AL) 19 April 1994 (1994-04-19) <br> * column 1, lines 9-14 * <br> * column 7, lines 8-12 * <br> * column 8, lines 28-38 * <br> * figure 3 * <br> * claim 4 * <br> ----- | 1-22 | INV. <br> A61N5/06 <br><br> ADD. <br> A61N5/067 |
| X | US 2019/126061 A1 (HONG SUNGHO [KR] ET AL) 2 May 2019 (2019-05-02) <br><br> * paragraphs [0072] - [0109] * <br> * figures 3-5 * <br> ----- | 1-4, 6-14, 16-22 | |
| X | WO 2010/045973 A1 (UNILEVER PLC [GB]; UNILEVER NV [NL] ET AL.) 29 April 2010 (2010-04-29) <br> * claims * <br> * figures * <br> ----- | 1-22 | |
| X | US 2007/150030 A1 (PEARL HENRY [US] ET AL) 28 June 2007 (2007-06-28) <br><br> * paragraphs [0060] - [0066] * <br> * figures 12-15 * <br> ----- | 1-4, 6-14, 16-22 | TECHNICAL FIELDS SEARCHED (IPC) <br><br> A61N |

The present search report has been drawn up for all claims

| Place of search | Date of completion of the search | Examiner |
|---|---|---|
| Munich | 7 October 2020 | Lohmann, Stefan |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

                                                                      
& : member of the same patent family, corresponding
  document

EPO FORM 1503 03.82 (P04C01)

**ANNEX TO THE EUROPEAN SEARCH REPORT
ON EUROPEAN PATENT APPLICATION NO.**

EP 20 17 6304

This annex lists the patent family members relating to the patent documents cited in the above-mentioned European search report.
The members are as contained in the European Patent Office EDP file on
The European Patent Office is in no way liable for these particulars which are merely given for the purpose of information.

07-10-2020

| Patent document cited in search report | | Publication date | Patent family member(s) | | Publication date |
|---|---|---|---|---|---|
| US 5303722 | A | 19-04-1994 | BR | 9006986 A | 24-12-1991 |
| | | | EP | 0451261 A1 | 16-10-1991 |
| | | | ES | 2042465 T1 | 16-12-1993 |
| | | | JP | 2893217 B2 | 17-05-1999 |
| | | | JP | H04502630 A | 14-05-1992 |
| | | | US | 5246019 A | 21-09-1993 |
| | | | US | 5303722 A | 19-04-1994 |
| | | | WO | 9106279 A2 | 16-05-1991 |
| US 2019126061 | A1 | 02-05-2019 | KR | 20190048383 A | 09-05-2019 |
| | | | US | 2019126061 A1 | 02-05-2019 |
| | | | WO | 2019088356 A1 | 09-05-2019 |
| WO 2010045973 | A1 | 29-04-2010 | BR | PI0822843 A2 | 23-06-2015 |
| | | | CN | 102196838 A | 21-09-2011 |
| | | | EA | 201170594 A1 | 31-10-2011 |
| | | | EP | 2349477 A1 | 03-08-2011 |
| | | | JP | 5603872 B2 | 08-10-2014 |
| | | | JP | 2012506266 A | 15-03-2012 |
| | | | WO | 2010045973 A1 | 29-04-2010 |
| | | | ZA | 201102725 B | 27-06-2012 |
| US 2007150030 | A1 | 28-06-2007 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• EP 18172363 A